# EUROPEAN PATENT APPLICATION

(11) **EP 1 232 752 A1**
(43) Date of publication of application: **21.08.2002**
(21) Application number: 01965291.6
(22) Date of filing: 05.09.2001
(51) Int. Cl.: A61K 33/00, A61P 11/00, A61K 47/02

(54) **HELIUM-OXYGEN MIXTURE WITH THERAPEUTIC APPLICATION**

(30) Priority: 06.09.2000 ES 200002182
(71) Applicant: SOCIEDAD ESPANOLA DE CARBUROS METALICOS S.A., 08038 Barcelona (ES)
(72) Inventor: RUBIANES LOPEZ, Rafael, 28700 San Sebastian De Los Reyes (ES); PENA GARCIA, Javier, 28700 San Sebastian De Los Reyes (ES)
(74) Representative: Ponti Sales, Adelaida
(86) International application number: ES0100336
(87) International publication number: WO02020025

(57) **Abstract**

This patent refers to the use of a mixture of helium and oxygen for therapeutic purposes.

The invention provides an alternative, noninvasive way for treating patients with acute respiratory failure using a mixture of gases with a helium:oxygen ratio ranging from 10:90 to 70:30, preferably between 20:80 and 60:40.

Helium is a light gas that facilitates oxygen transport without difficulty to every part of the human body, thus ensuring that the limbs, the part of the body most affected by oxygen deficit, are oxygenated adequately.

## Description

### Field of Application

The present invention relates to a mixture of helium and oxygen used for therapeutic purposes. More specifically, the invention refers to the use of a mixture of helium and oxygen for the treatment of patients with acute respiratory failure.

### State of the Art

In the state of the art, particularly in the medical field, invasive ventilation techniques are being used for the treatment of patients with acute respiratory failure, with the resulting side effects of these techniques.

Thus, in patients with chronic obstructive pulmonary disease, the first measure considered is positive pressure ventilation through a nasal or face mask without endotracheal intubation using devices that act synchronously with the patient's spontaneous breathing or with additional inspiratory assistance. However, these measures often are insufficient and tracheal intubation and mechanical ventilation are necessary. The decision to begin this treatment is determined by the patient's clinical condition.

Therefore, many studies have been conducted to find alternative treatments in order to avoid the use of invasive ventilation in patients with acute respiratory failure. However, to date, the only treatment that has produced satisfactory results has been specifically the application of invasive ventilation by endotracheal intubation with suitable devices.

In another field of the technique, mixtures of oxygen and helium in specific proportions have been used to fill air tanks for scuba diving.

An expert in this field would not look so far afield from the technique for a solution to this problem.

Therefore, the current state of the art of this technique has no noninvasive technique for the treatment of patients with acute respiratory failure.

### Description of the Invention

The present invention was designed to provide an alternative, noninvasive way to treat patients with acute respiratory failure. To date, the studies conducted and the techniques used are based on invasive procedures, with the resulting side effects that these techniques can produce in patients who receive this treatment.

On the other hand, there is a notable difference between treating a patient with an invasive technique or noninvasive technique. Invasive techniques require surgical intervention, the availability of specific materials, and highly qualified personnel, which makes them very expensive. Noninvasive techniques are easily applied, do not require highly qualified personnel, and are unquestionably more comfortable for the patient being treated.

An objective of the present invention is a composition comprising a mixture of helium and oxygen gas suitable for use in the treatment of acute respiratory failure by noninvasive ventilation.

Specially, the use of a mixture of helium and oxygen gases is designed especially for the treatment of patients with hypercapnia, which occurs when there is an excessive amount of carbon dioxide in blood.

Helium is a very light gas that can transport oxygen without difficulty to every part of the human body, thus ensuring that the limbs, which are the areas most affected by an oxygen deficit, are oxygenated adequately and preventing problems that can derive from insufficient oxygenation.

It is important to note the high diffusiveness of helium gas compared with nitrogen, a gas which has been used in the state of the art prior to this patent application.

The use of a mixture of helium and oxygen permits laminar flow of the gas supply. It also reduces airway resistance and obstruction to flow, thus increasing alveolar circulation and, consequently, improving the related muscle function.

Advantageously, the use of this gas mixture ranges from 10:90 to 70:30, preferably 20:80 to 60:40.

The helium and oxygen gas mixture can also contain a medicinal agent, such as a bronchodilator agent, that can act at the same time as treatment with the helium oxygen gas mixture.

### Results

The comparative values of noninvasive mechanical ventilation (NIMV) with air and noninvasive mechanical ventilation with the mixture of helium and oxygen gas described in this invention are given below.

| | NIMV with air | NIMV with He/O | |
|---|---|---|---|
| IPAP*:EPAP**, cm H₂O | 15.9±4.8: | 17.7±8; | ns |
| | 7±2.5 | 7.28±2.5 | |
| Respiratory rate | 25.4±6.9 | 23.6±3 | ns |
| Inspiratory flow (1/min) | 2.120±0.6 | 1.82±0.78 | sig |
| Expiratory flow (1/min) | 0.38±0.32 | 0.46±0.19 | sig |
| Loss (1/min) | 19.8±21 | 13.4±18 | ns |
| pH | 7.29±8.1 | 7.31± | sig |
| pCO₂ in blood | 80.16±24 | 75.98±24 | sig |
| Insp:exp time ratio (s) | 0.68±0.10 | 0.42±0.10 | sig |
| Inspiratory O₂ fraction | 0.50±9.34 | 0.97±2.3 | sig |

| | | | |
|---|---|---|---|
| * Inspiratory positive airway pressure. | | | |
| ** Expiratory positive airway pressure. Notes: pH of healthy patients = 7.35, ns = non-significant, pCO₂ of healthy patients = 45 mmHg, sig = significant | | | |

It was observed that the addition of a mixture of helium and oxygen gas obtained from a bottle of pure helium met safety requirements during noninvasive mechanical ventilation. An improvement in acidosis and respiratory parameters was achieved with the treatment of hypercapnic patients using a gas mixture of helium and oxygen administered by mask.

## Claims

1. Composition That comprises a helium and oxygen gas mixture for use in the treatment of acute respiratory failure by noninvasive ventilation.

2. Composition that comprises a gas mixture, as claimed in Claim 1, with a helium:oxygen ratio of the mixture ranging from 10:90 to 70:30, preferably from 20:80 to 60:40.

3. Composition that comprises a gas mixture, as claimed in Claims 1 or 2, in which the mixture also contains a medicinal agent.

4. Composition that comprises a gas mixture, as claimed in any of the above claims, and said mixture comprises., also, a bronchodilator.

5. Use of a mixture of helium and oxygen gas as claimed in any of the above claims for the preparation of a composition comprising this mixture for the treatment of hypercapnia.
